# EUROPEAN PATENT APPLICATION

(11) **EP 4 578 950 A1**
(43) Date of publication of application: **02.07.2025**
(21) Application number: 23857271.3
(22) Date of filing: 17.08.2023
(51) Int. Cl.: C12N 15/53, C12N 1/15, C12N 1/19, C12N 1/21, C12N 5/10, C12N 9/02, C12P 7/00, H01M 4/90, H01M 8/1004, H01M 8/1016, H01M 8/16

(54) **PROTEIN HAVING LACCASE ACTIVITY**

(30) Priority: 22.08.2022 JP 2022132106
(71) Applicant: Nagase & Co., Ltd., Osaka 550-8668 (JP)
(72) Inventor: KONO, Takunari, Kobe-shi, Hyogo 651-2241 (JP); UZURA, Atsuko, Kobe-shi, Hyogo 651-2241 (JP); UMEDA, Mana, Kobe-shi, Hyogo 651-2241 (JP)
(74) Representative: J A Kemp LLP
(86) International application number: PCT/JP2023/029678
(87) International publication number: WO 2024/043159

(57) **Abstract**

Provided is a new CueO mutant with improved activity compared with wild-type CueO. The protein according to one or more embodiments of the present invention comprises an amino acid sequence [1] or [2] and has laccase activity: [1] an amino acid sequence containing at least a region of positions 29-516 in SEQ ID NO: 14 with mutations (a) and (b): (a) a substitution of D360 with an amino acid other than D; and (b) one or more selected from a substitution of G304 with an amino acid other than G, a substitution of D373 with an amino acid other than D, and a substitution of Q374 with an amino acid other than Q; or [2] an amino acid sequence 90% or more identical to the sequence [1], and having residues corresponding to positions 360, 304, 373, and 374 of SEQ ID NO: 14 identical to those of the sequence [1].

GGAAGTACCGGGTGAAGTCGACGGCGGCCCGCAGGGAATTATTCCGCCAGG
TGGCAAGCGCTCGGTGACGTTGAACGTTGATCAACCTGCCGCTACCTGCTGG
TTCCATCCGCATCAGCACGGCAAAACCGGGCGACAGGTGGCGATGGGGCTG
GCTGGGCTGGTGGTGATTGAAGATGACGAGATCCTGAAATTAATGCTGCCAA
AACAGTGGGGTATCGATGATGTTCCGGTGATCGTTCAGGATAAGAAATTTAGC
GCCGACGGGCAGATTGATTATCAACTGGATGTGATGACCGCCGCCGTGGGCT
GGTTTGGCGATACGTTGCTGACCAACGGTGCAATCTACCCGCAACACGCTGC
CCCGCGTGGTTGGCTGCGCCTGCGTTTGCTCAATGGCTGTAATGCCCGTTCGC
TCAATTTCGCCACCAGCGACAATCGCCCGCTGTATGTGATTGCCAGCGACGG
TGGTCTGCTACCTGAACCAGTGAAGGTGAGCGAACTGCCGGTGCTGATGGG
CGAGCGTTTTGAAGTGCTGGTGGAGGTTAACGATAACAAACCCTTTGACCTG
GTGACGCTGCCGGTCAGCCAGATGGGGATGGCGATTGCGCCGTTTGATAAGC
CTCATCCGGTAATGCGGATTCAGCCGATTGCTATTAGTGCCTCCGGTGCTTTG
CCAGACACATTAAGTAGCCTGCCTGCGTTACCTTCGCTGGAAGGGCTGACGG
TACGCAAGCTGCAACTCTCTATGGACCCGATGCTCGATATGATGGGGATGCAG
ATGCTAATGGAGAAATATGGCGATCAGGCGATGGCCGGGATGGATCACAGCC
AGATGATGGGCCATATGGGGCACGGCAATATGAATCATATGAACCACGGCGG
GAAGTTCGATTTCCACCATGCCAACAAAATCAACGGTCAGGCGTTTGATATG
AACAAGCCGATGTTTGCGGCGGCGAAAGGGCAATACGAACGTTGGGTTATCT
CTGGCGTGGGCGACATGATGCTGCATCCGTTCCATATCCACGGCACGCAGTTC
CGTATCTTGTCAGAAAATGGCAAACCGCCAGCGGCTCATCGCGCGGGCTGGA
AAGATACCGTTAAGGTAGAAGGTAATGTCAGCGAAGTGCTGGTGAAGTTTAA
TCACGATGCACCGAAAGAACATGCTTATATGGCGCACTGCCATCTGCTGGAG
CATGAAGATACGGGGATGATGTTAGGGTTTACGGTATAA

## Description

### TECHNICAL FIELD

One or more embodiments of the present invention relate to a protein having laccase activity, a polynucleotide comprising a base sequence encoding the protein, a vector or a DNA fragment comprising the polynucleotide, and a transformed cell retaining the vector or the DNA fragment.

One or more embodiments of the present invention also relate to a method for oxidizing a laccase substrate compound using the protein.

One or more embodiments of the present invention also relate to a biofuel cell containing the protein.

### BACKGROUND ART

CueO, which is a copper efflux oxidase, derived from Escherichia coli is a multicopper oxidase having laccase activity.

Multicopper oxidases having laccase activity are used in various chemical reactions and industrial fields. For example, the multicopper oxidases are expected to be used in many industrial fields, such as treatment of effluents containing phenolic compounds and aromatic amines, removal of lignin in pulp manufacturing treatment and the like, production of electrode catalysts and artificial lacquer, synthesis of organic compounds, browning treatment of tea, reduction of bitterness in food and beverage products, production of gelatinizing agents for food products and cosmetic raw materials such as hair dyes, and use as clinical test reagents and bleaching agents. In addition, the multicopper oxidases are known to provide electrodes with a function as electron donors, and by combining them with suitable electrode substrates such as carbon substrates, the multicopper oxidases are expected to be used as electrode catalysts for cathodes (oxygen reduction electrodes) in biofuel cells and as electrode catalysts for biosensors (for example, Patent Document 1).

CueO contains a plurality of copper ions coordinated at the active center.

Non-Patent Document 1 discloses that the laccase activity of CueO (yacK) decreases with time when no copper ions are added, whereas the activity is sustained by adding copper ions.

Non-Patent Document 2 discloses that the fifth copper ion plays an important role in the electron transfer activity of CueO. Non-Patent Document 2 discloses that the electron transfer activity of mutants with an amino acid substitution introduced into one residue of the four amino acid residues M335, D360, D439, and M441 coordinated to the fifth copper ion in the amino acid sequence of wild-type CueO was examined and that 11 CueO mutants, such as D360K, significantly improved the electron transfer activity compared with the wild type.

Non-Patent Document 3 discloses that G304 in the amino acid sequence of wild-type CueO (G276 when the signal peptide composed of 28 amino acids at the N-terminal is excluded) plays an important role in the laccase activity of CueO and that CueO mutants with this residue substituted with K, R, N, and Y have significantly higher laccase activity compared with wild-type CueO, with the G304K mutant in particular having laccase activity 2.7 times that of the wild type.

Non-Patent Document 4 is a report by a researcher in the same group as Non-Patent Document 3. Non-Patent Document 4 discloses that in the G304K mutant with high laccase activity, the substrate is more easily accessible to type 1 copper ions due to conformational changes in met-rich (MR) helix and regulatory loop (R-loop) in the presence of copper ions, and that a triple mutant with D373A and Q374A mutations further introduced into the G304K mutant has even higher laccase activity than the G304K mutant.

Patent Document 1: JP-A-2014-3970

Non-Patent Document 1: Kim, C. et al., Journal of bacteriology, 183, 4866-4875 (2001)
Non-Patent Document 2: Zhang, L. et al., Chem. Eur. J. 26, 4974-7979 (2020)
Non-Patent Document 3: Yue, Q. et al., Bioresour. Bioprocess, 4, 48 (2017)
Non-Patent Document 4: Wang, H. et al., Sci. Rep. 8, 14252 (2018)

### DISCLOSURE OF THE INVENTION

### PROBLEMS TO BE SOLVED BY THE INVENTION

Non-Patent Document 1 suggests that the copper ions coordinated to CueO easily dissociate.

As disclosed in Non-Patent Documents 2, 3, and 4, it has been conventionally required to enhance the activity of CueO.

Therefore, an objective of the present invention is to provide a new CueO mutant with improved activity compared with wild-type CueO.

### SOLUTIONS TO THE PROBLEMS

The following embodiments are disclosed herein as one or more embodiments of the invention.
<1> A protein comprising an amino acid sequence [1] or [2]:
   [1] an amino acid sequence containing at least a region from position 29 to position 516 in an amino acid sequence of SEQ ID NO: 14 with mutations (a) and (b) introduced thereinto (an amino acid sequence represented by any one of SEQ ID NOs: 17, 18, and 19) :
      (a) a substitution of D at position 360 with an amino acid other than D; and
      (b) one or more selected from a substitution of G at position 304 with an amino acid other than G, a substitution of D at position 373 with an amino acid other than D, and a substitution of Q at position 374 with an amino acid other than Q; or
   [2] an amino acid sequence having 90% or more sequence identity to the amino acid sequence [1], the amino acid sequence having amino acid residues corresponding to positions 360, 304, 373, and 374 of the amino acid sequence of SEQ ID NO: 14 identical to those of the amino acid sequence [1], wherein
      the protein having laccase activity.
<2> The protein according to <1>, in which the mutation (a) is a substitution of D at position 360 with K or S, and the mutation (b) includes a substitution of G at position 304 with K, N, R, V, L, S, Y, C, D, F, P, T, A, or H.
<3> The protein according to <2>, in which the mutation (a) is a substitution of D at position 360 with K, and the mutation (b) includes a substitution of G at position 304 with K, N, R, V, L, S, Y, C, D, or F.
<4> The protein according to <3>, in which the mutation (b) includes a substitution of G at position 304 with K, N, or R.
<5> The protein according to <2>, in which the mutation (a) is a substitution of D at position 360 with S, and the mutation (b) includes a substitution of G at position 304 with V, P, T, N, R, H, A, L, F, C, or D.
<6> The protein according to <5>, in which the mutation (b) includes a substitution of G at position 304 with V, P, T, N, R, or H.
<7> The protein according to any one of <2> to <6>, in which the mutation (b) further includes a substitution of D at position 373 with A and a substitution of Q at position 374 with A.
<8> The protein according to any one of <1> to <7>, in which the amino acid sequence [2] includes a region identical to a region corresponding to positions 299 to position 398 of the amino acid sequence of SEQ ID NO: 14 in the amino acid sequence [1].
<9> The protein according to any one of <1> to <8>, comprising the amino acid sequence [1].
<10> The protein according to <9>, in which the mutation (a) is a substitution of D at position 360 with K or S, and the mutation (b) includes a substitution of G at position 304 with K, N, R, V, L, S, Y, C, D, F, P, T, A, or H.
<11> The protein according to <10>, in which the mutation (a) is a substitution of D at position 360 with K, and the mutation (b) includes a substitution of G at position 304 with K, N, R, V, L, S, Y, C, D, or F.
<12> The protein according to <11>, in which the mutation (b) includes a substitution of G at position 304 with K, N, or R.
<13> The protein according to <10>, in which the mutation (a) is a substitution of D at position 360 with S, and the mutation (b) includes a substitution of G at position 304 with V, P, T, N, R, H, A, L, F, C, or D.
<14> The protein according to <13>, in which the mutation (b) includes a substitution of G at position 304 with V, P, T, N, R, or H.
<15> The protein according to any one of <10> to <14>, in which the mutation (b) further includes a substitution of D at position 373 with A and a substitution of Q at position 374 with A.
<16> A polynucleotide comprising a base sequence encoding the amino acid sequence of the protein according to any one of <1> to <15>.
<17> A vector or a DNA fragment comprising the polynucleotide according to <16>.
<18> A transformed cell retaining the vector or the DNA fragment according to <17>.
<19> A method for oxidizing a laccase substrate compound, comprising oxidizing the laccase substrate compound in a reaction solution containing the protein according to any one of <1> to <15> and the laccase substrate compound.
<20> The method according to <19> not comprising adding copper ions to the reaction solution.
<21> A biofuel cell comprising a cathode-side electrode containing the protein according to any one of <1> to <15>.
<22> The biofuel cell according to <21>, further comprising an anode-side electrode, and an electrolyte layer arranged between the cathode-side electrode and the anode-side electrode, copper ions being not added to the electrolyte layer.

This description encompasses content disclosed in Japanese Patent Application No. 2022-132106 forming the basis for priority of this application.

All the publications, patents, and patent applications cited in the description are directly incorporated herein by reference.

### EFFECTS OF THE INVENTION

The protein according to one or more embodiments of the present invention has higher laccase activity than wild-type CueO and maintains laccase activity without adding copper ions.

The polynucleotide, the vector, the DNA fragment, and the transformed cell according to one or more embodiments of the present invention are useful for producing the protein.

With the method for oxidizing a laccase substrate compound according to one or more embodiments of the present invention, a laccase substrate compound can be oxidized using the protein.

The biofuel cell according to one or more embodiments of the present invention can be used for various applications.

### BRIEF DESCRIPTION OF THE DRAWINGS

Fig. 1 is a diagram describing primers for producing mut1 and mut2 in Example 1 and DNA fragments amplified by means of PCR using the primers.
Fig. 2 is a diagram describing primers for producing mut3 in Example 1 and a DNA fragment amplified by means of PCR using the primers.
Fig. 3 is a photograph showing a culture supernatant fluid (S), a filtrate (F), an ammonium sulfate-fractionated enzyme solution (A), and a purified enzyme solution (D) for enzyme WT, mut1, mut2, and mut3 produced by each of recombinant Escherichia coli BL21 (pKL-CueO), BL21 (pKL-CueO1), BL21 (pKL-CueO2), and BL21 (pKL-CueO3) produced in Example 1, being subjected to SDS-PAGE.
Fig. 4A is a graph showing the residual activity of ABTS oxidation activity for WT, mut1, mut2, and mut3 preserved at 60°C.
Fig. 4B is a graph showing the residual activity of ABTS oxidation activity for WT, mut1, mut2, and mut3 preserved at 70°C.
Fig. 4C is a graph showing the residual activity of ABTS oxidation activity for WT, mut1, mut2, and mut3 preserved at 80°C.
Fig. 5A is a graph showing the residual activity of ABTS oxidation activity for WT preserved at 4°C, 25°C, and 40°C for seven days.
Fig. 5B is a graph showing the residual activity of ABTS oxidation activity for mut1 preserved at 4°C, 25°C, and 40°C for seven days.
Fig. 5C is a graph showing the residual activity of ABTS oxidation activity for mut2 preserved at 4°C, 25°C, and 40°C for seven days.
Fig. 5D is a graph showing the residual activity of ABTS oxidation activity for mut3 preserved at 4°C, 25°C, and 40°C for seven days.
Fig. 6 illustrates a preferable example of a biofuel cell including a cathode-side electrode containing a mutant protein according to one embodiment of the present invention.

### DESCRIPTION OF PREFERRED EMBODIMENTS

### <Protein>

The protein according to one or more embodiments of the present invention contains an amino acid sequence [1] or [2] described above and has laccase (EC: 1.10.3.2) activity.

### (Laccase Activity)

Laccase activity is substrate oxidation activity and refers to the activity of oxidizing a laccase substrate compound selected from 2,2'-azino-bis(3-ethylbenzothiazoline-6-sulfonic acid) (hereinafter abbreviated as "ABTS"), 2,6-dimethoxyphenol, N-ethyl-N-(2-hydroxy-3-sulfopropyl)-3-methylaniline (hereinafter abbreviated as "TOOS"), dimethylaniline, diethylaniline, N,N-dimethyl-p-phenylenediamine, catechol, resorcinol, hydroquinone, phenol, guaiacol, pyrogallol, p-hydroxybenzoic acid, caffeic acid, hydrocaffeic acid, o-cresol, p-toluidine, o-chlorophenol, m-chlorophenol, p-chlorophenol, 2,4-dichlorophenol, 2,6-dichlorophenol, 2,4,6-trichlorophenol, p-phenylenediamine, propyl gallate, and the like. It especially refers to the activity of oxidizing ABTS.

Although the magnitude of the laccase activity of the protein according to this embodiment is not particularly limited, the protein preferably has higher laccase activity than laccase activity of a protein composed of position 29 to position 516 in the amino acid sequence shown in SEQ ID NO: 14 (wild-type CueO). The laccase activity for comparison with that of wild-type CueO is preferably ABTS oxidation activity per mg of protein (specific activity) measured by means of a measuring method described in Experiment 5 of Example 1, where no copper ions were added to the reaction system during the measurement. The specific activity of the protein according to this embodiment by means of the measuring method is, for example, 5.0 times or more, preferably 7.0 times or more, more preferably 10.0 times or more, and most preferably 13 times or more, that of wild-type CueO measured under the same conditions. The upper limit is not particularly limited, but it is, for example, 50 times or less. That is, the specific activity of the protein according to this embodiment by means of the measuring method can be 5.0 times or more and 50 times or less the specific activity of wild-type CueO measured under the same conditions, preferably within a further specified range specified by the above lower-limit and/or upper-limit values. A specific value of the specific activity of the protein according to this embodiment by means of the measuring method is, for example, 5,000 U/mg or more, preferably 7,000 U/mg or more, more preferably 10,000 U/mg or more, and most preferably 13,000 U/mg or more. The upper limit is not particularly limited, but it is, for example, 50,000 U/mg or less. That is, the specific activity of the protein according to this embodiment can be 5,000 U/mg or more and 50,000 U/mg or less, preferably within a further specified range specified by the above lower-limit and/or upper-limit values. Here, as the unit (U) of activity, the amount of enzyme that oxidizes 1 µmol of ABTS per minute was employed.

There has been a problem in that copper ions easily dissociate from wild-type CueO, and laccase activity decreases under conditions with no copper ions around. In contrast, the protein has an advantageous effect in which copper ions are difficult to dissociate from the protein according to this embodiment, and the protein retains high activity even in an environment with no copper ions around. For example, the protein according to this embodiment purified and recovered from a culture obtained by culturing transformed cells described below in a medium containing a sufficient amount of copper ions (for example, as CuSO₄) has high activity even in an environment with no copper ions around. The protein according to this embodiment having laccase activity refers to a protein (holoenzyme) retaining copper ions, unless otherwise limited. The protein according to this embodiment may be a protein retaining copper ions, which is obtained by producing a protein (apoenzyme) that does not contain copper ions through expression and then incubating the protein (apoenzyme) in a buffer solution containing copper ions.

The protein according to this embodiment is preferably a protein that retains copper ions and is purified or crude purified.

### (Amino Acid Sequence [1])

The amino acid sequence [1] is an amino acid sequence containing at least a region from position 29 to position 516 in the amino acid sequence of SEQ ID NO: 14, which is of wild-type CueO derived from Escherichia coli, with mutations (a) and (b) introduced thereinto (an amino acid sequence represented by any one of SEQ ID NOs: 17, 18, and 19). The mutation (a) is a substitution of D at position 360 with an amino acid other than D. The mutation (b) is one or more selected from a substitution of G at position 304 with an amino acid other than G, a substitution of D at position 373 with an amino acid other than D, and a substitution of Q at position 374 with an amino acid other than Q.

Amino acid sequence of SEQ ID NO: 14:
MQRRDFLKYSVALGVASALPLWSRAVFAAERPTLPIPDLLTTDARNRIQLTIGAG
QSTFGGKTATTWGYNGNLLGPAVKLQRGKAVTVDIYNQLTEETTLHWHGLEVP
GEVDGGPQGIIPPGGKRSVTLNVDQPAATCWFHPHQHGKTGRQVAMGLAGLVV
IEDDEILKLMLPKQWGIDDVPVIVQDKKFSADGQIDYQLDVMTAAVGWFGDTL
LTNGAIYPQHAAPRGWLRLRLLNGCNARSLNFATSDNRPLYVIASDGGLLPEPV
KVSELPVLMGERFEVLVEVNDNKPFDLVTLPVSQMGMAIAPFDKPHPVMRIQPI
AISASGALPDTLSSLPALPSLEGLTVRKLQLSMDPMLDMMGMQMLMEKYGDQ
AMAGMDHSQMMGHMGHGNMNHMNHGGKFDFHHANKINGQAFDMNKPMFA
AAKGQYERWVISGVGDMMLHPFHIHGTQFRILSENGKPPAAHRAGWKDTVKV
EGNVSEVLVKFNHDAPKEHAYMAHCHLLEHEDTGMMLGFTV

Position 1 to position 28 of the amino acid sequence of SEQ ID NO: 14 constitute a signal sequence, which is not directly involved in the laccase activity of the protein. Therefore, the amino acid sequence [1] may be the whole of the amino acid sequence of SEQ ID NO: 14 with the mutations (a) and (b) introduced thereinto (the amino acid sequence represented by any one of SEQ ID NOs: 17, 18, and 19). Alternatively, the amino acid sequence [1] may be an amino acid sequence composed only of the region from position 29 to position 516 in the amino acid sequence of SEQ ID NO: 14 with the mutations (a) and (b) introduced thereinto. Alternatively, the amino acid sequence [1] may be an amino acid sequence composed of a region of position N (N is an integer between 2 and 28) to position 516 in the amino acid sequence of SEQ ID NO: 14 with the mutations (a) and (b) introduced thereinto.

As used herein, with the "amino acid," an optically active amino acid refers to an L-amino acid unless otherwise specified.

In Examples, it has been confirmed that CueO mutants with both the mutation (a) and the mutation (b) introduced have significantly higher laccase activity under a condition where copper ions are not added compared with that of wild-type CueO.

In (a) described above, the "amino acid other than D" is preferably K or S, and more preferably K. That is, the mutation (a) is preferably D360K (a substitution of D at position 360 with K) or D360S (a substitution of D at position 360 with S), and more preferably D360K. Hereinafter, amino acid substitution mutations may be indicated in the same way.

It is only necessary for the mutation (b) to be one or more selected from the "substitution of G at position 304 with an amino acid other than G," the "substitution of D at position 373 with an amino acid other than D," and the "substitution of Q at position 374 with an amino acid other than Q," and the mutation (b) may be any combination of one to three thereof. The mutation (b) is preferably composed only of the "substitution of G at position 304 with an amino acid other than G," or a combination of the "substitution of G at position 304 with an amino acid other than G" and one or more of the "substitution of D at position 373 with an amino acid other than D" and the "substitution of Q at position 374 with an amino acid other than Q," and further preferably a combination of the "substitution of G at position 304 with an amino acid other than G," the "substitution of D at position 373 with an amino acid other than D," and the "substitution of Q at position 374 with an amino acid other than Q."

The mutation (b) preferably includes at least the "substitution of G at position 304 with an amino acid other than G," and more preferably includes the substitution of G at position 304 with K, N, R, V, L, S, Y, C, D, F, P, T, A, or H. In these cases, the mutation (a) is particularly preferably D360K or D360S.

When the mutation (a) is D360K, the mutation (b) preferably includes at least the "substitution of G at position 304 with an amino acid other than G," more preferably includes the substitution of G at position 304 with K, N, R, V, L, S, Y, C, or D, and most preferably includes the substitution of G at position 304 with K, N, or R.

When the mutation (a) is D360S, the mutation (b) preferably includes at least the "substitution of G at position 304 with an amino acid other than G," more preferably includes the substitution of G at position 304 with V, P, T, N, R, H, A, L, F, C, or D, and most preferably includes the substitution of G at position 304 with V, P, T, N, R, or H.

In the mutation (b), the "substitution of D at position 373 with an amino acid other than D" is preferably D373A, and the "substitution of Q at position 374 with an amino acid other than Q" is preferably Q374A.

Examples of a particularly preferable combination of the mutation (a) and the mutation (b) include one or more selected from combination numbers 1 to 112 shown in the table below. From the viewpoint of the laccase activity level, as the combination of the mutation (a) and the mutation (b), one or more selected from combination numbers 1 to 10, 16 to 19, 22 to 28, 29 to 38, 44 to 47, 50 to 56, 57 to 66, 72 to 75, 78 to 84, 85 to 94, 100 to 103, and 106 to 112 are particularly preferable. One or more selected from combination numbers 1 to 3, 16 to 18, 25, 26, 28, 29 to 31, 44 to 46, 53, 54, 56, 57 to 59, 72 to 74, 81, 82, 84, 85 to 87, 100 to 102, 109, 110, and 112 are further preferable. One or more selected from combination numbers 1 to 3, 16 to 18, 25, 26, 28, and 85 are still more preferable. Combination numbers 1 to 3 and 85 are most preferable. SEQ ID NO: 20 shows amino acid sequences containing the signal peptide with any of the mutations under the combination numbers 1 to 112 introduced into the amino acid sequence shown in SEQ ID NO: 14.

**[Table 1]**

| Combination number | Mutation (a) | Mutation (b) |
|---|---|---|
| 1 | D360K | G304K |
| 2 | D360K | G304N |
| 3 | D360K | G304R |
| 4 | D360K | G304V |
| 5 | D360K | G304L |
| 6 | D360K | G304S |
| 7 | D360K | G304Y |
| 8 | D360K | G304C |
| 9 | D360K | G304D |
| 10 | D360K | G304F |
| 11 | D360K | G304P |
| 12 | D360K | G304T |
| 13 | D360K | G304A |
| 14 | D360K | G304H |
| | | |
| 15 | D360S | G304K |
| 16 | D360S | G304N |
| 17 | D360S | G304R |
| 18 | D360S | G304V |
| 19 | D360S | G304L |
| 20 | D360S | G304S |
| 21 | D360S | G304Y |
| 22 | D360S | G304C |
| 23 | D360S | G304D |
| 24 | D360S | G304F |
| 25 | D360S | G304P |
| 26 | D360S | G304T |
| 27 | D360S | G304A |
| 28 | D360S | G304H |
| | | |
| 29 | D360K | G304K+D373A |
| 30 | D360K | G304N+D373A |
| 31 | D360K | G304R+D373A |
| 32 | D360K | G304V+D373A |
| 33 | D360K | G304L+D373A |
| 34 | D360K | G304S+D373A |
| 35 | D360K | G304Y+D373A |
| 36 | D360K | G304C+D373A |
| 37 | D360K | G304D+D373A |
| 38 | D360K | G304F+D373A |
| 39 | D360K | G304P+D373A |
| 40 | D360K | G304T+D373A |
| 41 | D360K | G304A+D373A |
| 42 | D360K | G304H+D373A |
| | | |
| 43 | D360S | G304K+D373A |
| 44 | D360S | G304N+D373A |
| 45 | D360S | G304R+D373A |
| 46 | D360S | G304V+D373A |
| 47 | D360S | G304L+D373A |
| 48 | D360S | G304S+D373A |
| 49 | D360S | G304Y+D373A |
| 50 | D360S | G304C+D373A |
| 51 | D360S | G304D+D373A |
| 52 | D360S | G304F+D373A |
| 53 | D360S | G304P+D373A |
| 54 | D360S | G304T+D373A |
| 55 | D360S | G304A+D373A |
| 56 | D360S | G304H+D373A |
| | | |
| 57 | D360K | G304K+Q374A |
| 58 | D360K | G304N+Q374A |
| 59 | D360K | G304R+Q374A |
| 60 | D360K | G304V+Q374A |
| 61 | D360K | G304L+Q374A |
| 62 | D360K | G304S+Q374A |
| 63 | D360K | G304Y+Q374A |
| 64 | D360K | G304C+Q374A |
| 65 | D360K | G304D+Q374A |
| 66 | D360K | G304F+Q374A |
| 67 | D360K | G304P+Q374A |
| 68 | D360K | G304T+Q374A |
| 69 | D360K | G304A+Q374A |
| 70 | D360K | G304H+Q374A |
| | | |
| 71 | D360S | G304K+Q374A |
| 72 | D360S | G304N+Q374A |
| 73 | D360S | G304R+Q374A |
| 74 | D360S | G304V+Q374A |
| 75 | D360S | G304L+Q374A |
| 76 | D360S | G304S+Q374A |
| 77 | D360S | G304Y+Q374A |
| 78 | D360S | G304C+Q374A |
| 79 | D360S | G304D+Q374A |
| 80 | D360S | G304F+Q374A |
| 81 | D360S | G304P+Q374A |
| 82 | D360S | G304T+Q374A |
| 83 | D360S | G304A+Q374A |
| 84 | D360S | G304H+Q374A |
| | | |
| 85 | D360K | G304K+D373A+Q374A |
| 86 | D360K | G304N+D373A+Q374A |
| 87 | D360K | G304R+D373A+Q374A |
| 88 | D360K | G304V+D373A+Q374A |
| 89 | D360K | G304L+D373A+Q374A |
| 90 | D360K | G304S+D373A+Q374A |
| 91 | D360K | G304Y+D373A+Q374A |
| 92 | D360K | G304C+D373A+Q374A |
| 93 | D360K | G304D+D373A+Q374A |
| 94 | D360K | G304F+D373A+Q374A |
| 95 | D360K | G304P+D373A+Q374A |
| 96 | D360K | G304T+D373A+Q374A |
| 97 | D360K | G304A+D373A+Q374A |
| 98 | D360K | G304H+D373A+Q374A |
| | | |
| 99 | D360S | G304K+D373A+Q374A |
| 100 | D360S | G304N+D373A+Q374A |
| 101 | D360S | G304R+D373A+Q374A |
| 102 | D360S | G304V+D373A+Q374A |
| 103 | D360S | G304L+D373A+Q374A |
| 104 | D360S | G304S+D373A+Q374A |
| 105 | D360S | G304Y+D373A+Q374A |
| 106 | D360S | G304C+D373A+Q374A |
| 107 | D360S | G304D+D373A+Q374A |
| 108 | D360S | G304F+D373A+Q374A |
| 109 | D360S | G304P+D373A+Q374A |
| 110 | D360S | G304T+D373A+Q374A |
| 111 | D360S | G304A+D373A+Q374A |
| 112 | D360S | G304H+D373A+Q374A |

### (Amino Acid Sequence [2])

The amino acid sequence [2] has a "sequence identity" of preferably 92% or more, more preferably 94% or more, more preferably 95% or more, more preferably 96% or more, more preferably 97% or more, more preferably 98% or more, and more preferably 99% or more. However, the "sequence identity" in the amino acid sequence [2] is less than 100%.

The "sequence identity" in the amino acid sequence [2] refers to the ratio (%) of identical amino acid residues to the total amino acid residue count of the amino acid sequence [1] when the amino acid sequence [2] is aligned (brought into alignment) with the amino acid sequence [1] and gaps are introduced as necessary to ensure that the degree of amino acid match between both sequences is the highest. The sequence identity can be calculated using a protein search system such as BLAST or FASTA (Karlin, S. et al., 1993, Proc. Natl. Acad. Sci. USA, 90: 5873-5877; Altschul, S. F. et al., 1990, J. Mol. Biol., 215: 403-410; Pearson, W. R. et al., 1988, Proc. Natl. Acad. Sci. USA, 85: 2444-2448).

The "amino acid residues corresponding to positions 360, 304, 373, and 374 of the amino acid sequence of SEQ ID NO: 14" in the amino acid sequence [2] refer to amino acid residues in the amino acid sequence [2], which correspond to the amino acid residues in the amino acid sequence [1] corresponding to positions 360, 304, 373, and 374 of the amino acid sequence of SEQ ID NO: 14 when the amino acid sequence [2] is aligned with the amino acid sequence [1]. That is, the amino acid residues, which correspond to positions 360, 304, 373, and 374 of the amino acid sequence of SEQ ID NO: 14, in the amino acid sequence [1] are also preserved in the amino acid sequence [2].

The amino acid sequence [2] preferably includes a region identical to the region in the amino acid sequence [1] corresponding to preferably from position 299 to position 398, more preferably from position 250 to position 450, more preferably from position 250 to position 500, more preferably from position 250 to position 510, more preferably from position 100 to position 510, and more preferably from position 30 to position 510, of the amino acid sequence of SEQ ID NO: 14. When these regions are identical, the amino acid sequence [2] is preferable because it is particularly highly likely to have the same activity as the amino acid sequence [1].

### <Polynucleotide Comprising Base Sequence Encoding Amino Acid Sequence of Mutant Protein>

One or more embodiments of the present invention also relate to a polynucleotide comprising a base sequence encoding the amino acid sequence of the protein according to the above embodiment (hereinafter referred to as the "mutant protein disclosed herein" in some cases). The mutant protein disclosed herein can be produced using various mutation introduction techniques known in this technical field. For example, the mutant protein disclosed herein can be produced by mutating a polynucleotide encoding an amino acid residue to be modified in a wild-type CueO gene that encodes its reference amino acid sequence into a polynucleotide encoding the amino acid residue after the modification, and then expressing the protein from the mutated gene.

The polynucleotide according to this embodiment may contain single-stranded or double-stranded DNA, cDNA, RNA, or other artificial nucleic acids. The DNA, cDNA, and RNA may be chemically synthesized. The polynucleotide may also contain a nucleotide sequence of an untranslated region (UTR) in addition to an open reading frame (ORF). In addition, the polynucleotide according to this embodiment may be codon-optimized in accordance with the organism species of a transformed cell that retains a vector or a DNA fragment comprising the polynucleotide. Information on codons used by various living organisms is available from the Codon Usage Database ([www.kazusa.or.jp/codon/]).

As means for mutating amino acid residues in the amino acid sequence of wild-type CueO, various mutation introduction techniques known in this technical field can be used. For example, in a polynucleotide comprising a base sequence encoding the amino acid sequence of wild-type CueO (hereinafter also referred to as a parent gene), by mutating a base sequence encoding an amino acid residue, which should be mutated, into a base sequence encoding an amino acid residue after the mutation, a polynucleotide comprising the base sequence encoding the amino acid sequence of the mutant protein disclosed herein can be obtained.

The introduction of a desired mutation into the parent gene can basically be performed using various site-directed mutagenesis methods well-known to those skilled in the art. For example, a site-directed mutagenesis method can be performed by any method, such as the inverse PCR method or annealing method. Commercially available site-directed mutagenesis kits (such as QuickChange II Site-Directed Mutagenesis Kit and QuickChange Multi Site-Directed Mutagenesis Kit from Stratagene) can also be used.

Site-directed mutagenesis to a parent gene can most commonly be performed using a mutation primer containing a nucleotide mutation to be introduced. It is only necessary to design the mutation primer to be annealed to a region containing a base sequence encoding an amino acid residue to be mutated in a parent gene and to have a base sequence containing a base sequence (codon) encoding an amino acid residue after the mutation instead of the base sequence (codon) encoding the amino acid residue to be mutated. The base sequences (codons) encoding the amino acid residues before and after the mutation can be recognized and selected appropriately by those skilled in the art based on ordinary textbooks and the like. Alternatively, for site-directed mutagenesis, a method for linking DNA fragments, which are obtained by separately using two complementary primers containing a nucleotide mutation to be introduced to amplify the respective upstream and downstream sides of the mutation site, into one by means of SOE (splicing by overlap extension)-PCR (Gene, 1989, 77(1): pp. 61-68) can be used.

A template DNA containing a parent gene can be prepared by extracting genomic DNA from microorganisms that produce CueO by means of the standard method. Alternatively, based on the amino acid sequence of wild-type CueO, the corresponding base sequence may be chemically synthesized and used as the template DNA.

A mutation primer can be produced by means of a well-known oligonucleotide synthesis method, such as the phosphoramidite method (Nucleic Acids Research, 1989, 17: 7059-7071). Such primer synthesis can also be performed using, for example, a commercially available oligonucleotide synthesizer (such as the one manufactured by Applied Biosystems, Inc.). By using a primer set including the mutation primers and performing the site-directed mutagenesis described above with the parent gene as the template DNA, the polynucleotide according to this embodiment having desired mutations can be obtained.

### <Vector or DNA Fragment>

One or more embodiments of the present invention also relate to a vector or a DNA fragment that comprises the polynucleotide comprising a base sequence encoding the amino acid sequence of the mutant protein disclosed herein.

The type of the vector according to this embodiment is not particularly limited, and the vector may be any vector, such as plasmid, phage, phagemid, cosmid, virus, YAC vector, and shuttle vector. In addition, the vector according to this embodiment is, but not limited to, preferably a vector capable of replicating in bacteria, preferably in Escherichia coli, and more preferably an expression vector capable of inducing the expression of transgenes in Escherichia coli. The vector according to this embodiment may be a plasmid vector derived from Escherichia coli (such as pET22b(+), pBR322, pBR325, pUC57, pUC118, pUC119, pUC18, pUC19, and pBluescript) into which the polynucleotide comprising a base sequence encoding the amino acid sequence of the mutant protein disclosed herein is incorporated.

The vector or the DNA fragment according to this embodiment may include a DNA replication initiation region or a DNA region containing a replication origin. Alternatively, in the vector or the DNA fragment according to this embodiment, a regulatory sequence, such as a promoter region for initiating the transcription of the gene, a terminator region, or a secretory signal region for secreting the protein produced through expression out of the cell may be operably linked with the polynucleotide comprising a base sequence encoding the amino acid sequence of the mutant protein disclosed herein (that is, a mutant gene). Note that a gene and a regulatory sequence being "operably linked" means that a gene and a regulatory region are arranged such that the gene can be expressed under the regulation of the regulatory region.

The type of the regulatory sequence, such as the promoter region, terminator region, and secretory signal region, described above is not particularly limited, and the normally used promoter sequence, terminator sequence, or secretory signal sequence can be appropriately selected and used according to the host into which it is to be introduced.

Alternatively, a marker gene (for example, a resistance gene for drugs such as ampicillin, neomycin, kanamycin, and chloramphenicol) for selecting the host into which the vector has been properly introduced may be further incorporated into the vector or the DNA fragment according to this embodiment. Alternatively, when an auxotroph is used as the host, a gene encoding a required nutritional synthesis enzyme may be incorporated into the vector as a marker gene. Further, alternatively, when a selective medium that requires a specific metabolism for growth is used, a gene related to the metabolism may be incorporated into the vector as a marker gene.

Linking the polynucleotide comprising a base sequence encoding the amino acid sequence of the mutant protein disclosed herein with the regulatory sequence and the marker gene can be performed by means of a method known in the field, such as the SOE-PCR method. The procedure for introducing the linked fragments into the vector is well-known in the field.

### <Transformed Cell>

One or more embodiments of the present invention also relate to a transformed cell that retains the vector or the DNA fragment that comprises the polynucleotide comprising a base sequence encoding the amino acid sequence of the mutant protein disclosed herein. The transformed cell according to this embodiment can be obtained by introducing the vector into a host cell or by introducing the DNA fragment into the genome of a host cell. The transformed cell retains the vector or the DNA fragment such that the mutant protein disclosed herein can be produced through expression.

Host cells include microorganisms, such as bacteria, filamentous fungi, and yeast. Examples of bacteria include Escherichia coli and bacteria belonging to Staphylococcus, Enterococcus, and Bacillus, among which Escherichia coli is preferred. Examples of filamentous fungi include Trichoderma, Aspergillus, and Rhizopus. Examples of yeast include Pichia, Saccharomyces, and Schizosaccharomyces.

As a method for introducing the vector into a host cell, the methods normally used in the field, such as the protoplast method and electroporation method, can be used. A desired transformant with the vector introduced can be obtained by selecting a strain that has undergone introduction properly using the expression of a marker gene, auxotrophy, and the like as indicators.

Alternatively, the transformed cell according to this embodiment can be obtained by introducing the DNA fragment that comprises the polynucleotide comprising a base sequence encoding the amino acid sequence of the mutant protein disclosed herein directly into the genome of a host cell. For example, by means of the SOE-PCR method or the like, the polynucleotide, a regulatory sequence, and a marker gene sequence are linked to form a fragment, and base sequences complementary to the genome of the host cell are added to both ends of the fragment to construct a DNA fragment. Then, the introduction of the DNA fragment into the host cell and causing homologous recombination between the genome of the host cell and the DNA fragment introduces the DNA fragment comprising the polynucleotide into the genome of the host cell.

The transformed cell according to this embodiment obtained in this way, into which the vector or the DNA fragment that comprises the polynucleotide comprising a base sequence encoding the amino acid sequence of the mutant protein disclosed herein has been introduced, is cultured in an appropriate medium, and accordingly, the mutant gene on the vector or the DNA fragment is expressed to produce the mutant protein disclosed herein. The medium used for the culture of the transformed cell according to this embodiment can be selected appropriately by those skilled in the art according to the type of microorganism of the transformant.

Alternatively, the mutant protein disclosed herein may be produced through the expression of the polynucleotide comprising a base sequence encoding the amino acid sequence of the mutant protein disclosed herein or its transcript using a cell-free translation system. The "cell-free translation system" is an in vitro transcription-translation system or an in vitro translation system configured by adding a reagent such as amino acids necessary for protein translation to a suspension obtained by mechanically disrupting a cell to be a host.

The mutant protein disclosed herein produced in the culture of the transformed cell according to this embodiment or in the cell-free translation system described above can be isolated or purified using standard methods used for protein purification, such as centrifugation, ammonium sulfate precipitation, gel chromatography, ion-exchange chromatography, and affinity chromatography, either individually or in appropriate combinations. At this time, when the gene encoding the amino acid sequence of the mutant protein disclosed herein is operably linked to the secretory signal sequence on the vector or the DNA fragment in the transformed cell, the produced protein can be more easily recovered from the culture because it is secreted out of the cell. The protein recovered from the culture may be further purified by a known means.

The mutant protein disclosed herein as an expression product can be obtained by culturing the transformed cell according to this embodiment and collecting it from the culture. The "culture" means any of a culture supernatant, a cultured cell, or a crushed object of a cultured cell. As a method for culturing the transformed cell, an ordinary method used for culturing host cells can be followed.

As a medium for culturing the transformed cell according to this embodiment, a medium containing copper ions is preferably used. Copper ions can be added to a medium, for example, as CuSO₄ or its hydrate. The mutant protein disclosed herein, which has been obtained from the culture of the transformed cell according to this embodiment cultured in a medium containing copper ions, retains copper ions necessary for activity in a stable state.

The medium for culturing the transformed cell according to this embodiment can further contain carbon sources, nitrogen sources, inorganic salts, and the like that are assimilable by a host cell. As the medium, any natural media or synthetic media may be used. The carbon sources include carbohydrates, such as glucose, galactose, fructose, xylose, sucrose, raffinose, and starch; organic acids, such as acetic acid and propionic acid; and alcohols, such as ethanol and propanol. The nitrogen sources include ammonia, ammonium salts of inorganic acids or organic acids, such as ammonium chloride, ammonium sulfate, ammonium acetate, and ammonium phosphate, or other nitrogen-containing compounds. In addition, peptone, meat extracts, corn steep liquor, and various amino acids and the like may be used. Inorganic substances include monopotassium phosphate, dipotassium phosphate, magnesium phosphate, magnesium sulfate, sodium chloride, ferrous sulfate, manganese sulfate, copper sulfate, calcium carbonate, and the like. Further, if necessary, antifoaming agents, such as vegetable oil, surfactants, and silicon may be added.

It is only necessary to appropriately select the culture conditions according to the type of medium, a culture method, and the like. The culture conditions are not particularly limited as long as the conditions allow the host cell to grow and produce the mutant protein disclosed herein. Further, while the type of culturing is not particularly limited, a liquid medium can be preferably used from the viewpoint of mass culture.

When the mutant protein disclosed herein accumulates in the transformed cell, the transformed cell is recovered by centrifugation after the culture is finished. After the resulting transformed cell is crushed by sonication and the like, a cell-free extract is obtained by centrifugation and the like. Using this as starting material, the mutant protein disclosed herein can be purified by means of standard protein purification methods, such as the salting-out method and various kinds of chromatography, including ion-exchange chromatography, gel filtration chromatography, hydrophobic chromatography, and affinity chromatography. When the mutant protein disclosed herein, which has been produced through expression, is secreted outside of the transformed cell, it can be similarly purified from the culture supernatant.

### <Method for Oxidizing Laccase Substrate Compound>

Still another one or more embodiments of the present invention relate to a method for oxidizing a laccase substrate compound, which includes, in a reaction solution containing the mutant protein disclosed herein and the laccase substrate compound, oxidizing the laccase substrate compound.

Here, examples of the laccase substrate compound include the compounds described above, and ABTS is particularly preferable.

The reaction solution can contain water as a solvent, preferably a buffered aqueous solution regulated to an appropriate pH range, for example, a range of 4.0 to 10.0.

The reaction of oxidizing the laccase substrate compound with the mutant protein disclosed herein can be performed under a temperature condition of, for example, 10°C to 40°C, and more preferably 25°C to 35°C.

The method according to this embodiment preferably does not include adding copper ions to the reaction solution. Surprisingly, the inventors found that the mutant protein disclosed herein retained laccase activity even without the addition of copper ions, and instead, that the laccase activity was improved when no copper ions were added. It is considered that the mutant protein disclosed herein retains copper ions beforehand and can stably retain the copper ions even in the reaction solution where copper ions are not added.

### <Biofuel Cell>

Still another one or more embodiments of the present invention relate to a biofuel cell including a cathode-side electrode containing the mutant protein disclosed herein.

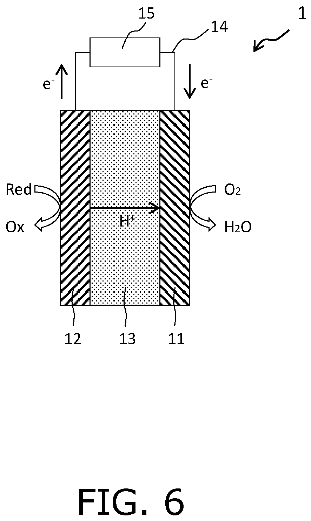

Fig. 6 illustrates an example of the biofuel cell according to this embodiment. A biofuel cell 1 illustrated in Fig. 6 includes a cathode-side electrode 11 containing the mutant protein disclosed herein, an anode-side electrode 12, and an electrolyte layer 13 arranged between the cathode-side electrode 11 and the anode-side electrode 12. The cathode-side electrode 11 and the anode-side electrode 12 are electrically connected by an external circuit 14, and a load 15 is arranged on the external circuit 14. At the anode-side electrode 12, a reductant Red of a substrate (fuel) is oxidized to an oxidized product Ox. Electrons generated by this reaction reach the cathode-side electrode 11 via the external circuit 14 including the load 15 to generate an electric current, and protons generated by the reaction reach the cathode-side electrode 11 via the electrolyte layer 13. At the cathode-side electrode 11, the activity of the mutant protein disclosed herein causes the electrons, the protons, and oxygen in the air to react, generating water. The electrolyte layer 13 in the biofuel cell 1 according to this embodiment is preferably an electrolyte layer to which copper ions are not added. As described above, the mutant protein disclosed herein retains laccase activity even without the addition of copper ions, and instead, the laccase activity is higher when no copper ions are added. The biofuel cell according to this embodiment can achieve even higher performance by using an electrolyte layer to which copper ions are not added.

The cathode-side electrode 11 in the biofuel cell 1 according to this embodiment is preferably an electrode in which the mutant protein disclosed herein is immobilized on a conductive substrate (for example, a porous carbon electrode) connected to the external circuit 14. In addition, the mutant protein disclosed herein may be supplied to a conductive substrate in the form of being dissolved in an appropriate buffer solution. For immobilization, the mutant protein disclosed herein is preferably immobilized in a state of a holoenzyme containing copper ions. However, the mutant protein disclosed herein may be immobilized in the form of an apoenzyme, and copper ions may be supplied as another layer or in the form of being dissolved in an appropriate buffer solution.

The anode-side electrode 12 can be an electrode in which an enzyme with the activity to oxidize the reductant Red of the substrate (fuel) to the oxidized product Ox is immobilized on a conductive substrate (for example, a porous carbon electrode). Reducing substances, such as sugars, alcohols, organic acids, amines, hydrogen, and other inorganic compounds, which living organisms can utilize as energy sources, can be used as the reductant Red of the substrate (fuel).

As for the electrolyte layer 13, as long as it has ionic conductivity capable of transmitting protons and the like and has the property of not transmitting anode-side components other than ions such as the protons, cathode-side components, or the electrons, there are no limitations on the material, shape, and the like of the electrolyte layer 13. For example, a solid electrolyte membrane can be used and configured as a diaphragm. Examples of the solid electrolyte membrane include solid membranes and the like having an ion-exchange function of organic polymers having strong acid groups, such as sulfonic acid groups, phosphate groups, phosphonic acid groups, and phosphinic acid groups; weak acid groups, such as carboxyl groups; and polar groups. In addition, substances necessary for the expression of the catalytic activity of an enzyme may be supplied as another layer or in the form of being dissolved in an appropriate buffer solution.

In the cathode-side electrode 11 of the biofuel cell 1 according to this embodiment, the mutant protein disclosed herein catalyzes the reaction of receiving the electrons from the anode-side electrode and reducing oxygen to water. Since the mutant protein disclosed herein has excellent catalytic ability with high laccase activity, the biofuel cell 1 according to this embodiment containing the mutant protein disclosed herein has high efficiency in redox reaction on the cathode-side electrode 11. As a result, the electric energy obtained increases, enabling high output power and stable electric power generation.

### <Other Applications>

The mutant protein disclosed herein has excellent catalytic ability, such as improved laccase activity (specific activity). Therefore, the mutant protein disclosed herein can be applied to technologies that require redox reactions of phenolic compounds in various industrial fields, such as medical, food, and environmental fields, in addition to the electrochemistry field.

### EXAMPLES

### <Example 1>

### (Experiment 1: Construction of Plasmid pKL223)

Using a plasmid pTrcHis (product name, manufactured by Invitrogen) as a template, PCR was performed with primers Bsa-lacI-forward (SEQ ID NO: 1) and Bam-lacI-reverse (SEQ ID NO: 2) to amplify the region containing the lacI^{q} gene and its regulatory promoter. KOD -plus- (product name, manufactured by TOYOBO Co., Ltd.) was used to perform PCR under the following reaction conditions: Step 1: 94°C, 2 minutes; Step 2: 94°C, 15 seconds; Step 3: 62°C, 30 seconds; Step 4: 68°C, 1 minute; Step 5: 4°C, ∞; with Step 2 through Step 4 repeated for 30 cycles. The resulting amplified fragment of approximately 1400 bp was double-digested with BsaAI and BamHI, and the double-digested fragment was inserted into the BsaAI-BamHI gap located upstream of the tac promoter of a plasmid pKK223-3 (product name, manufactured by GE HealthCare Japan) to obtain a plasmid pKL223. The sequences of the primers used are shown below.
Bsa-lacI-forward:5'-TATCGCTACGTGACTGGGTCATGGCT-3' (SEQ ID NO: 1)
Bam-lacI-reverse:5'-CGGGATCCTCGCGCTAACTCACATTAATTGCGTTGC-3' (SEQ ID NO: 2)

### (Experiment 2: Construction of Plasmid for Expression of CueO)

Based on the amino acid sequence of CueO derived from Escherichia coli (UniProtKB/Swiss-Prot Accession No. P36649, SEQ ID NO: 14), the corresponding DNA sequence registered in the database (CueO gene, GenBank Accession No. AP009048.1:137083-138633, SEQ ID NO: 3) was searched. A PCR primer EcoRI-CueO-F (SEQ ID NO: 5) with the WSD sequence (AGGAGGTATTAT, SEQ ID NO: 4) added immediately upstream of the initiation codon and a PCR primer HindIII-CueO-R (SEQ ID NO: 6) were designed. Using the designed two primers, PCR was performed with chromosomal DNA of the Escherichia coli W3110 (NBRC12713) strain prepared with QIAGEN Genomic-tip and Genomic DNA Buffer Set (product names, manufactured by Qiagen) as a template to amplify the CueO gene. PrimeSTAR (registered trademark) GXL DNA Polymerase (product name, manufactured by Takara Bio) was used to perform PCR under the following reaction conditions: Step 1: 98°C, 2 minutes; Step 2: 98°C, 10 seconds; Step 3: 68°C, 1 minute 30 seconds; Step 4: 12°C, ∞; with Step 2 through Step 3 repeated for 30 cycles. The resulting DNA fragment was digested with EcoRI and HindIII, and the digested DNA fragment was inserted into the EcoRI-HindIII gap located downstream of the tac promoter of the plasmid pKL223 to construct a plasmid pKL-CueO. Subsequently, the plasmid pKL-CueO was introduced into an Escherichia coli BL21 strain to obtain a transformed Escherichia coli (referred to as "recombinant Escherichia coli" in some cases) BL21 (pKL-CueO). The base sequences of the primers used are shown below.

EcoRI-CueO-F (SEQ ID NO: 5): 
5'-

AAAAGTTGAATTCAGGAGGTATTATATGCAACGTCGTGATTTCTTAAAATATTC

CG-3'
HindIII-CueO-R (SEQ ID NO: 6):
   5'-CCCAAGCTTTTATACCGTAAACCCTAACATCATCCCCGTATCTTC-3'

### Culture of Recombinant Escherichia coli BL21 (pKL-CueO)

Recombinant Escherichia coli BL21 (pKL-CueO) was cultured in 5 ml of LB Broth, Miller (product name, manufactured by Nacalai Tesque) with ampicillin added at a final concentration of 50 µg/ml by shaking it overnight at 200 rpm while heating it to 37°C to prepare a liquid seed culture. Subsequently, using a 500-ml baffled Erlenmeyer flask (produced by PYREX), 50 µL of the liquid seed culture was added to 2× LB Broth, Lennox (product name, manufactured by Nacalai Tesque) containing 50 ml CuSO₄·5H₂O (5 mM) and lactose (2%), and the culture was started at 37°C and 160 rpm. After 24 or 48 hours, cells were collected from the liquid culture by centrifugation (10,000×g for 15 minutes at 4°C).

### (Experiment 3: Production of CueO Mutant)

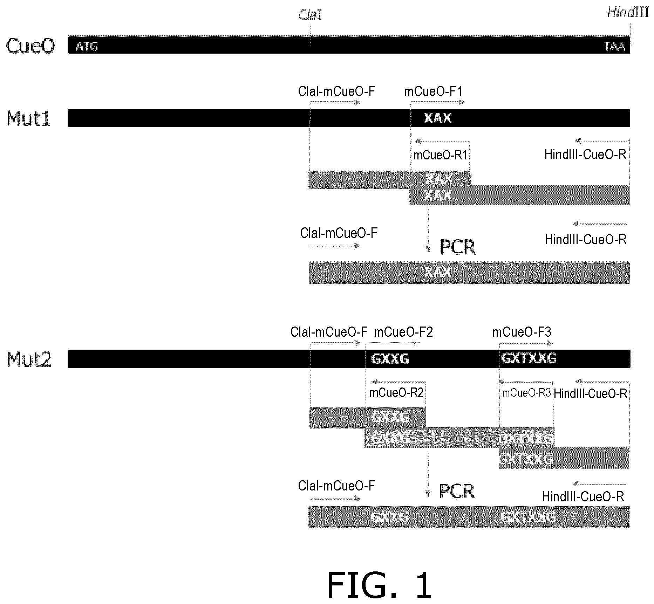

### Construction of Plasmid for Expression of D360K Mutant (mut1) (See the upper section of Fig. 1)

PCR primers ClaI-mCueO-F (SEQ ID NO: 7) and mCueO-R1 (SEQ ID NO: 8) and PCR primers mCueO-F1 (SEQ ID NO: 9) and HindIII-CueO-R (SEQ ID NO: 6) were designed. Using the designed primers, PCR was performed with pKL-CueO as a template to amplify two fragments. Next, using ClaI-mCueO-F (SEQ ID NO: 7) and HindIII-CueO-R (SEQ ID NO: 6), PCR was performed with a mixture of the two amplified fragments as a template to produce a DNA fragment with the mutated sequence for mutant CueO-D360K. The same conditions as in Experiment 2 were used for PCR. The resulting DNA fragment was digested with ClaI and HindIII, and the digested DNA fragment was inserted into the ClaI-HindIII gap of the plasmid pKL-CueO to construct a plasmid pKL-mCueO1. The base sequences of the primers used are shown below.

ClaI-mCueO-F (SEQ ID NO: 7):
   5'-TGGGGTATCGATGATGTTCCGGTGATCG-3'
mCueO-R1 (SEQ ID NO: 8): Underlined positions indicate introduced mutations.
   5'-GCATCCCCATCATTTTGAGCATCGGGTCC-3'
mCueO-F1 (SEQ ID NO: 9): Underlined positions indicate introduced mutations.
   5'-GGACCCGATGCTCAAAATGATGGGGATGC-3'
HindIII-CueO-R (SEQ ID NO: 6):
   5'-CCCAAGCTTTTATACCGTAAACCCTAACATCATCCCCGTATCTTC-3'

### Construction of Plasmid for Expression of G304K/D373A/Q374A Mutant (mut2) (See the lower section of Fig. 1)

PCR primers ClaI-mCueO-F (SEQ ID NO: 7) and mCueO-R2 (SEQ ID NO: 10), PCR primers mCueO-F2 (SEQ ID NO: 11) and mCueO-R3 (SEQ ID NO: 12), and PCR primers mCueO-F3 (SEQ ID NO: 13) and HindIII-CueO-R (SEQ ID NO: 6) were designed. Using the designed primers, PCR was performed with pKL-CueO as a template to amplify three fragments. Next, using ClaI-mCueO-F (SEQ ID NO: 7) and HindIII-CueO-R (SEQ ID NO: 6), PCR was performed with a mixture of the three amplified fragments as a template to produce a DNA fragment with the mutated sequence for mutant CueO-G304K/D373A/Q374A. The same conditions as in Experiment 2 were used for PCR. The resulting DNA fragment was digested with ClaI and HindIII, and the digested DNA fragment was inserted into the ClaI-HindIII gap of the plasmid pKL-CueO to construct a plasmid pKL-mCueO2. The base sequences of the primers used are shown below.

ClaI-mCueO-F (SEQ ID NO: 7):
   5'-TGGGGTATCGATGATGTTCCGGTGATCG-3'
mCueO-R2 (SEQ ID NO: 10): Underlined positions indicate introduced mutations.
   5'-GCAATCGCCATCTTCATCTGGCTGACC-3'
mCueO-F2 (SEQ ID NO: 11): Underlined positions indicate introduced mutations.
   5'-GGTCAGCCAGATGAAGATGGCGATTGC-3'
mCueO-R3 (SEQ ID NO: 12): Underlined positions indicate introduced mutations.
   5'-GGCCATCGCCGCAGCGCCATATTTC-3'
mCueO-F3 (SEQ ID NO: 13): Underlined positions indicate introduced mutations.
   5'-GAAATATGGCGCTGCGGCGATGGCC-3'
HindIII-CueO-R (SEQ ID NO: 6):
   5'-CCCAAGCTTTTATACCGTAAACCCTAACATCATCCCCGTATCTTC-3'

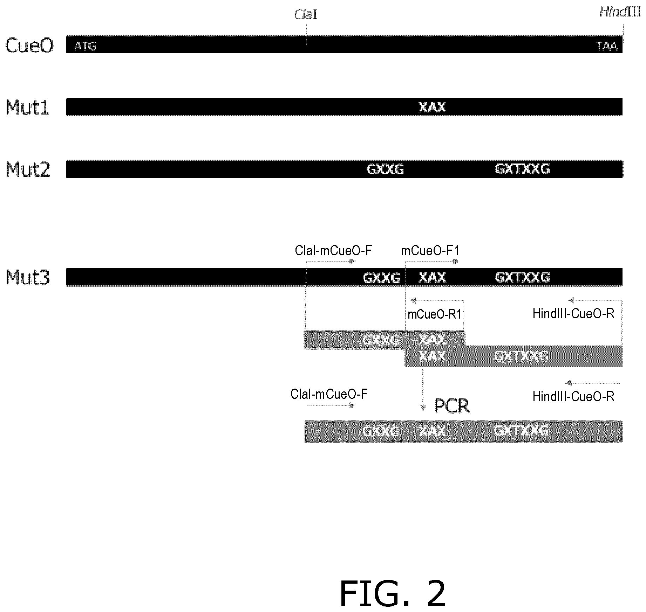

### Construction of Plasmid for Expression of G360K, G304K/D373A/Q374A Mutant (mut3) (See Fig. 2)

Using the primers designed for constructing the mutant mut1, PCR was performed with pKL-CueO2 as a template to amplify two fragments. Next, using ClaI-mCueO-F (SEQ ID NO: 7) and HindIII-CueO-R (SEQ ID NO: 6), PCR was performed with a mixture of the two amplified fragments as a template to produce a DNA fragment with the mutated sequence for mutant CueO-G360K, G304K/D373A/Q374A. The same conditions as in Experiment 2 were used for PCR. The resulting DNA fragment was digested with ClaI and HindIII, and the digested DNA fragment was inserted into the ClaI-HindIII gap of the plasmid pKL-CueO to construct a plasmid pKL-mCueO3. The base sequences of the primers used are shown below. The amino acid sequence of mut3 containing the signal peptide is shown in SEQ ID NO: 21.

ClaI-mCueO-F (SEQ ID NO: 7):
   5'-TGGGGTATCGATGATGTTCCGGTGATCG-3'
mCueO-R1 (SEQ ID NO: 8): Underlined positions indicate introduced mutations.
   5'-GCATCCCCATCATTTTGAGCATCGGGTCC-3'
mCueO-F1 (SEQ ID NO: 9): Underlined positions indicate introduced mutations.
   5'-GGACCCGATGCTCAAAATGATGGGGATGC-3'
HindIII-CueO-R (SEQ ID NO: 6):
   5'-CCCAAGCTTTTATACCGTAAACCCTAACATCATCCCCGTATCTTC-3'

CueO gene (SEQ ID NO: 3)

ATGCAACGTCGTGATTTCTTAAAATATTCCGTCGCGCTGGGTGTGGCTTCGGC

TTTGCCGCTGTGGAGCCGCGCAGTATTTGCGGCAGAACGCCCAACGTTACCG

ATCCCTGATTTGCTCACGACCGATGCCCGTAATCGCATTCAGTTAACTATTGGC

GCAGGCCAGTCCACCTTTGGCGGGAAAACTGCAACTACCTGGGGCTATAACG

GCAATCTGCTGGGGCCGGCGGTGAAATTACAGCGCGGCAAAGCGGTAACGG

TTGATATCTACAACCAACTGACGGAAGAGACAACGTTGCACTGGCACGGGCT

GGAAGTACCGGGTGAAGTCGACGGCGGCCCGCAGGGAATTATTCCGCCAGG
TGGCAAGCGCTCGGTGACGTTGAACGTTGATCAACCTGCCGCTACCTGCTGG
TTCCATCCGCATCAGCACGGCAAAACCGGGCGACAGGTGGCGATGGGGCTG
GCTGGGCTGGTGGTGATTGAAGATGACGAGATCCTGAAATTAATGCTGCCAA
AACAGTGGGGTATCGATGATGTTCCGGTGATCGTTCAGGATAAGAAATTTAGC
GCCGACGGGCAGATTGATTATCAACTGGATGTGATGACCGCCGCCGTGGGCT
GGTTTGGCGATACGTTGCTGACCAACGGTGCAATCTACCCGCAACACGCTGC
CCCGCGTGGTTGGCTGCGCCTGCGTTTGCTCAATGGCTGTAATGCCCGTTCGC
TCAATTTCGCCACCAGCGACAATCGCCCGCTGTATGTGATTGCCAGCGACGG
TGGTCTGCTACCTGAACCAGTGAAGGTGAGCGAACTGCCGGTGCTGATGGG
CGAGCGTTTTGAAGTGCTGGTGGAGGTTAACGATAACAAACCCTTTGACCTG
GTGACGCTGCCGGTCAGCCAGATGGGGATGGCGATTGCGCCGTTTGATAAGC
CTCATCCGGTAATGCGGATTCAGCCGATTGCTATTAGTGCCTCCGGTGCTTTG
CCAGACACATTAAGTAGCCTGCCTGCGTTACCTTCGCTGGAAGGGCTGACGG
TACGCAAGCTGCAACTCTCTATGGACCCGATGCTCGATATGATGGGGATGCAG
ATGCTAATGGAGAAATATGGCGATCAGGCGATGGCCGGGATGGATCACAGCC
AGATGATGGGCCATATGGGGCACGGCAATATGAATCATATGAACCACGGCGG
GAAGTTCGATTTCCACCATGCCAACAAAATCAACGGTCAGGCGTTTGATATG
AACAAGCCGATGTTTGCGGCGGCGAAAGGGCAATACGAACGTTGGGTTATCT
CTGGCGTGGGCGACATGATGCTGCATCCGTTCCATATCCACGGCACGCAGTTC
CGTATCTTGTCAGAAAATGGCAAACCGCCAGCGGCTCATCGCGCGGGCTGGA
AAGATACCGTTAAGGTAGAAGGTAATGTCAGCGAAGTGCTGGTGAAGTTTAA
TCACGATGCACCGAAAGAACATGCTTATATGGCGCACTGCCATCTGCTGGAG
CATGAAGATACGGGGATGATGTTAGGGTTTACGGTATAA

### (Experiment 4: Preparation of CueO Protein)

The plasmids pKL-mCueO1, pKL-mCueO2, and pKL-mCueO3 constructed in Experiment 3 were introduced into Escherichia coli BL21 strains to obtain recombinant Escherichia coli BL21 (pKL-CueO1), BL21 (pKL-CueO2), and BL21 (pKL-CueO3), respectively.

The recombinant Escherichia coli BL21 (pKL-CueO1), BL21 (pKL-CueO2), and BL21 (pKL-CueO3), and the recombinant Escherichia coli BL21 (pKL-CueO) prepared in Experiment 2 were stirred and cultured in 2× LB Broth, Lennox (product name, manufactured by Nacalai Tesque) containing 5 mM CuSO₄·5H₂O and 2% lactose at 30°C for 48 hours.

After the liquid culture was heat-treated at 62°C for two hours, a supernatant fluid (S) was recovered through ultrasonic disruption.

Calcium chloride was added to the supernatant fluid (S), and aggregates were removed by centrifugation (10,000×g for 30 minutes at 4°C). Afterward, the supernatant fluid was filtered with a 0.2 µm filter, and a filtrate (F) was recovered.

Ammonium sulfate was added to the filtrate (F) to form a 55% ammonium sulfate solution, which underwent salting-out. The precipitate was redissolved in deionized water to form an ammonium sulfate-fractionated enzyme solution (A).

The ammonium sulfate-fractionated enzyme solution (A) was dialyzed using 20 mM Tris-HCl (pH 7.6) buffer to perform buffer exchange, resulting in a purified enzyme solution (D).

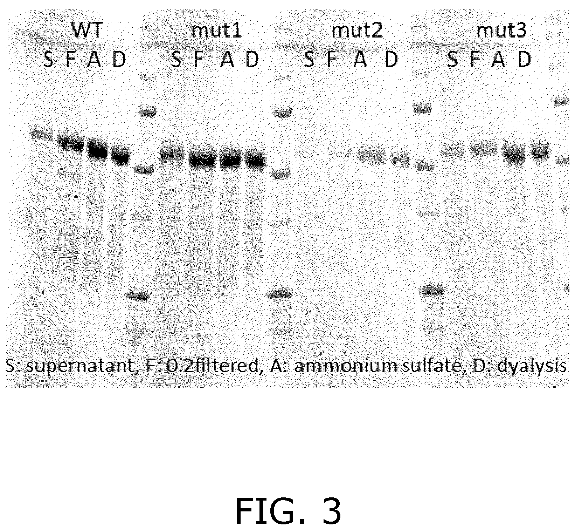

The supernatant fluid (S), the filtrate (F), the ammonium sulfate-fractionated enzyme solution (A), and the purified enzyme solution (D) for enzyme WT, mut1, mut2, and mut3 produced by each of the recombinant Escherichia coli BL21 (pKL-CueO), BL21 (pKL-CueO1), BL21 (pKL-CueO2), and BL21 (pKL-CueO3) were subjected to SDS (Sodium dodecyl sulfate)-polyacrylamide gel electrophoresis (SDS-PAGE). The results are shown in Fig. 3. In every case, a band was observed at a position corresponding to the molecular weight of CueO. It was also confirmed that the enzyme was purified in the purified enzyme solution (D).

### (Experiment 5: Measurement of ABTS Oxidation Activity)

ABTS oxidation activity (activity to oxidize ABTS (2,2'-azino-bis(3-ethylbenzothiazoline-6-sulfonic acid)) per mg of protein in the purified enzyme solutions of WT, mut1, mut2, and mut3 prepared in Experiment 4 was measured according to the following procedure.

Measuring method of the amount of protein in purified enzyme solution: The amount of protein of each purified enzyme solution was quantitatively determined using the Bradford method. The purified enzyme solution was well mixed with Quick Start Bradford 1× Dye Reagent (product name, manufactured by BIO-RAD), and the difference in absorbance at a wavelength of 595 nm with respect to a protein-free background was measured by a spectrophotometer. The protein concentration was determined from the standard straight line drawn from the absorbance of a standard sample (standard) with a known protein concentration.

The purified enzyme solution was diluted with a dilution buffer (20 mM Tris-HCl (pH 7.0), 0.02% Tween 20) to prepare a diluted enzyme solution. Under the condition of 30°C, using a solution with 10 mM ABTS dissolved in a 50 mM acetic acid buffer solution (pH 5.0) as a substrate solution, the diluted enzyme solution was added thereto in a multiwell plate, and the change in absorbance at 420 nm from 0 to 600 seconds later was measured using a spectrophotometer (Multiskan GO, produced by Thermo Fisher Scientific).

The activity value was calculated using a molar absorbance coefficient ε₄₂₀ = 36,000 for ABTS at 420 nm. As the unit of activity (U), the amount of enzyme oxidizing 1 µmol of ABTS per minute was used (Reference Document: JP-A-2010-183857).

The measurement results of the ABTS oxidation activity per mg of enzyme protein of WT, mut1, mut2, and mut3 and the ratio of the ABTS oxidation activity of mut1, mut2, and mut3 to that of WT are shown in the table below. The specific activity for the ABTS oxidation activity of mut3 was significantly high, reaching 14 times that of WT and mut1 and 2.1 times that of mut2.

**[Table 2]**

| | Mutation site | Specific activity (U/mg) | Fold (/WT) |
|---|---|---|---|
| WT | Wild type | 913 | 1.00 |
| mut1 | D360K | 1,096 | 1.20 |
| mut2 | G304K/D373A/Q374A | 6,207 | 6.80 |
| mut3 | D360K/G304K/D373A/Q374A | 13,163 | 14.42 |

### (Experiment 6: Effect of Copper Addition on ABTS Oxidation Activity)

The dilution buffer in Experiment 5 did not contain copper. The specific activity for the ABTS oxidation activity of WT, mut1, mut2, and mut3 was measured according to the same procedure as in Experiment 5, except that after the purified enzyme solution was diluted using 20 mM Tris-HCl (pH 7.0), 0.02% Tween 20, and 4 mM CuSO₄ as a dilution buffer and then incubated at 30°C for five minutes, a substrate was added thereto, to investigate the effect of copper addition on the ABTS oxidation activity of each enzyme.

The results are shown in the table below. Cu- indicates the specific activity in Experiment 5 using the dilution buffer without containing copper, and Cu+ indicates the specific activity in this experiment using the dilution buffer containing copper. In addition, for each enzyme, the ratio of the specific activity of Cu+ to the specific activity of Cu- is shown.

It was indicated that the addition of copper was necessary for the activation of WT. On the other hand, it was indicated that mut3 had high ABTS oxidation activity when no copper was added, and instead, its ABTS oxidation activity tended to be suppressed when copper was added.

**[Table 3]**

| | | Specific activity (U/mg) | | Fold |
|---|---|---|---|---|
| | Mutation site | Cu- | Cu+ | (Cu+/Cu-) |
| WT | Wild type | 913 | 9,723 | 10.65 |
| mut1 | D360K | 1,096 | 1,414 | 1.29 |
| mut2 | G304K/D373A/Q374A | 6,207 | 7,701 | 1.24 |
| mut3 | D360K + G304K/D373A/Q374A | 13,163 | 6,023 | 0.46 |

### (Experiment 7: Thermal Stability Test)

The specific activity for the ABTS oxidation activity of WT, mut1, mut2, and mut3 was measured according to the same procedure as in Experiment 5, except that a diluted enzyme solution, which was incubated at 60°C, 70°C, or 80°C after preparation, sampled every 15 minutes, and cooled on ice for 5 minutes, was used as the diluted enzyme solution.

The ratio of the specific activity measured using the diluted enzyme solution incubated at each temperature for a predetermined period to the specific activity measured using the diluted enzyme solution that was not incubated at each temperature was determined as residual activity (%).

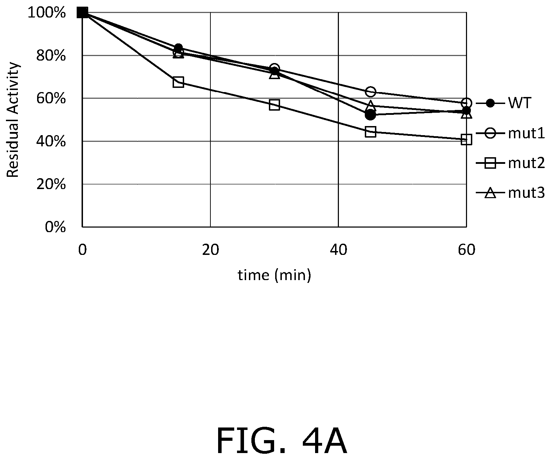

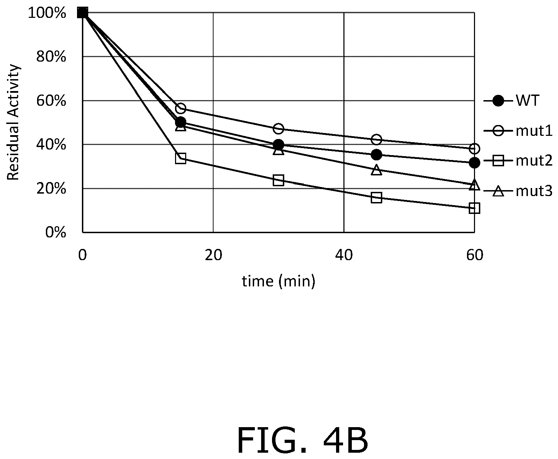

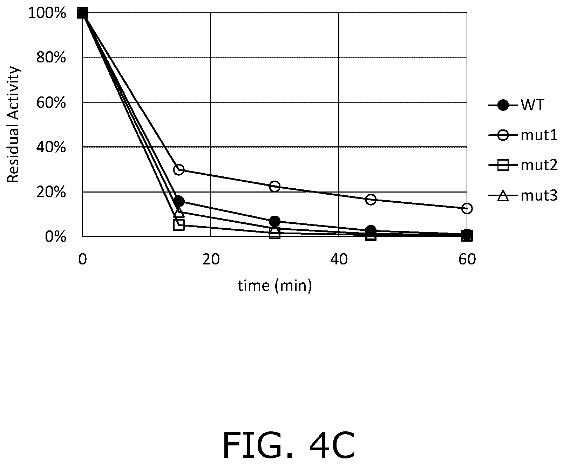

The results are shown in Fig. 4A (incubation temperature 60°C), Fig. 4B (incubation temperature 70°C), and Fig. 4C (incubation temperature 80°C). In terms of thermal stability, it was indicated that there was no substantial difference between mut3 and WT.

### (Experiment 8: Preservation Test)

The following procedure was used to prepare 60% glycerol products (liquid products) of WT, mut1, mut2, and mut3. Glycerol was added to each purified enzyme solution such that the weight ratio of each purified enzyme solution to glycerol was 4:6, and the mixture was determined as the 60% glycerol product (liquid product).

The 60% glycerol product of each enzyme was preserved at 4°C, 25°C, and 40°C for seven days. The specific activity for ABTS oxidation activity of samples before preservation and after seven-day preservation was measured according to the same procedure as in Experiment 5. The ratio of the specific activity after seven-day preservation at each temperature to the specific activity before the preservation at each temperature was determined as the residual activity (%).

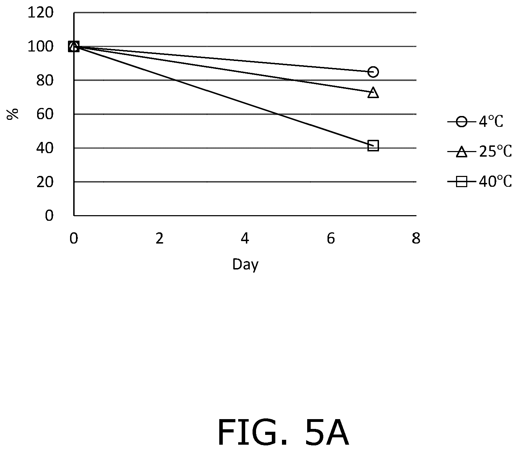

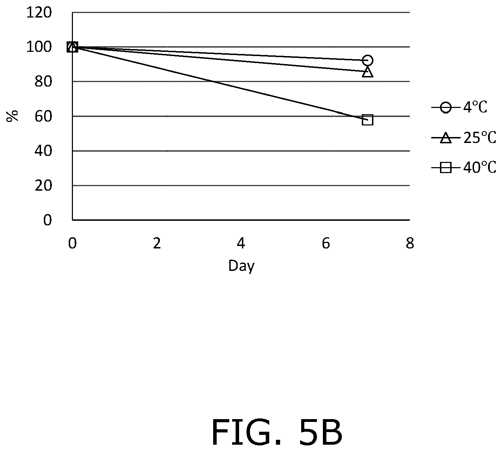

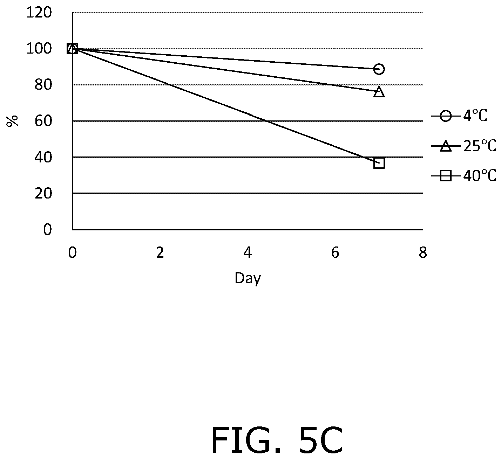

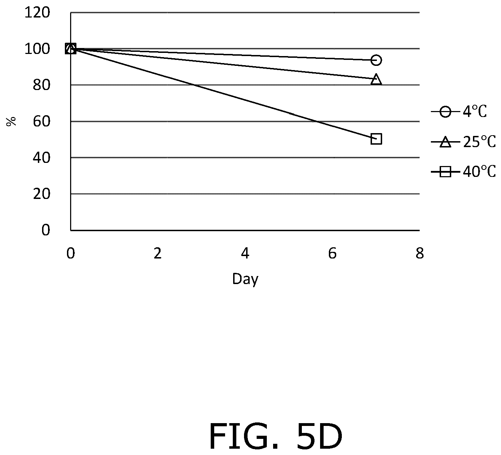

The results are shown in Fig. 5A (WT), Fig. 5B (mut1), Fig. 5C (mut2), and Fig. 5D (mut3). It was confirmed that the preservation stabilities of mut1 and mut3 were slightly improved compared with that of WT.

### <Example 2>

### (Experiment 1: Production of Plasmid for Expression of D360K/G304X Mutant)

Using the plasmid pKL-mCueO1 for expression of the D360K mutant (mut1) constructed in Experiment 3 of Example 1 as a template, plasmids with the mutation of G304X (X is K, N, R, V, L, S, Y, C, D, or F) introduced were constructed according to the following procedure.

When G304K was introduced, using the primers ClaI-mCueO-F (SEQ ID NO: 7) and mCueO-R2 (SEQ ID NO: 10) and primers mCueO-F2 (SEQ ID NO: 11) and HindIII-CueO-R (SEQ ID NO: 6) described in Experiment 3 of Example 1, PCR was performed with pKL-mCueO1 as a template to amplify two fragments. Next, using ClaI-mCueO-F (SEQ ID NO: 7) and HindIII-CueO-R (SEQ ID NO: 6), PCR was performed with a mixture of the two amplified fragments as a template to produce a DNA fragment with the mutated sequence for mutant CueO-D360K/G304K. The same conditions as in Experiment 2 of Example 1 were used for PCR. The resulting DNA fragment was digested with ClaI and HindIII, and the digested DNA fragment was inserted into the ClaI-HindIII gap of the plasmid pKL-CueO to construct a plasmid for expression of D360K/G304K mutant.

When G at position 304 was substituted with N, R, V, L, S, Y, C, D, or F, using the primers ClaI-mCueO-F (SEQ ID NO: 7) and mCueO-R2-N (SEQ ID NO: 15) and primers mCueO-F2-N (SEQ ID NO: 16) and HindIII-CueO-R (SEQ ID NO: 6), PCR was performed with pKL-mCueO1 as a template to amplify two fragments. Next, using ClaI-mCueO-F (SEQ ID NO: 7) and HindIII-CueO-R (SEQ ID NO: 6), PCR was performed with a mixture of the two amplified fragments as a template to produce a DNA fragment with the mutated sequence for mutant CueO-D360K/G304X. The same conditions as in Experiment 2 of Example 1 were used for PCR. The resulting DNA fragment was digested with ClaI and HindIII, and the digested DNA fragment was inserted into the ClaI-HindIII gap of the plasmid pKL-CueO to construct a plasmid for expression of D360K/G304X mutant.
ClaI-mCueO-F (SEQ ID NO: 7):
   5'-TGGGGTATCGATGATGTTCCGGTGATCG-3'
mCueO-R2-N (SEQ ID NO: 15) Underlined positions indicate introduced mutations (N is a mixture of A, T, G, and C):
   5'-GCAATCGCCATCNNCATCTGGCTGACC-3'
mCueO-F2-N (SEQ ID NO: 16) Underlined positions indicate introduced mutations (N is a mixture of A, T, G, and C):
   5'-GGTCAGCCAGATGNNGATGGCGATTGC-3'
HindIII-CueO-R (SEQ ID NO: 6):
   5'-CCCAAGCTTTTATACCGTAAACCCTAACATCATCCCCGTATCTTC-3'

Each plasmid constructed above was sequenced, and those confirmed to have the predetermined amino acid substitutions introduced were used in Experiment 2 below.

### (Experiment 2: Preparation of CueO Protein)

Each plasmid constructed and whose sequence was verified in Experiment 1 was introduced into an Escherichia coli BL21 strain to obtain recombinant Escherichia coli.

The recombinant Escherichia coli was stirred and cultured in 2× LB Broth, Lennox (product name, manufactured by Nacalai Tesque) containing 5 mM CuSO₄·5H₂O and 2% lactose at 30°C for 72 hours.

After the liquid culture was heat-treated at 62°C for two hours, it underwent ultrasonic disruption. The ultrasonically disrupted material was centrifuged, and the supernatant fluid was recovered.

The amount of protein in the supernatant fluid and the ABTS oxidation activity were measured according to the same procedure as in Experiment 5 of Example 1 to determine the specific activity.

For comparison, the recombinant Escherichia coli BL21 (pKL-CueO), BL21 (pKL-CueO1), BL21 (pKL-CueO2), and BL21 (pKL-CueO3) obtained in Example 1 were also cultured under the same conditions, and the liquid cultures were heat-treated and underwent ultrasonic disruption and centrifugation to obtain supernatant fluids. For the supernatant fluids, the amount of protein and the ABTS oxidation activity were measured in the same manner to determine the specific activity.

The following table shows the ratio of the specific activity of the supernatant fluid obtained from the recombinant Escherichia coli by each plasmid for mutant expression to the specific activity (WT) of the supernatant fluid obtained from the recombinant Escherichia coli BL21 (pKL-CueO).

All D360K/G304X double mutants were confirmed to have ABTS oxidation activity exceeding those of wild-type WT and mut1 (D360K). In particular, mut4 (D360K/G304K), mut9 (D360K/G304N), and mut12 (D360K/G304R) had even higher activity than mut3 (D360K/G304K/D373A/Q374A), which was confirmed to have high activity in Example 1. The amino acid sequences of mut4 to mut13 containing the signal peptides are shown in SEQ ID NO: 22.

**[Table 4]**

| | Mutation site | Fold (/WT) |
|---|---|---|
| WT | Wild type | 1.0 |
| mut1 | D360K | 0.5 |
| mut2 | G304K/D373A/Q374A | 2.2 |
| mut3 | D360K/G304K/D373A/Q374A | 9.1 |
| mut4 | D360K/G304K | 11.6 |
| mut5 | D360K/G304V | 4.8 |
| mut6 | D360K/G304L | 3.8 |
| mut7 | D360K/G304F | 1.8 |
| mut8 | D360K/G304S | 3.7 |
| mut9 | D360K/G304N | 12.4 |
| mut10 | D360K/G304Y | 3.2 |
| mut11 | D360K/G304C | 3.1 |
| mut12 | D360K/G304R | 10.5 |
| mut13 | D360K/G304D | 5.7 |

### <Example 3>

### (Experiment 1: Production of Plasmid for Expression of D360S/G304X Mutant)

### Construction of Plasmid for Expression of D360S Mutant

PCR primers ClaI-mCueO-F (SEQ ID NO: 7) and mCueO-R1S (SEQ ID NO: 24) and PCR primers mCueO-F1S (SEQ ID NO: 25) and HindIII-CueO-R (SEQ ID NO: 6) were designed. Using the designed primers, PCR was performed with pKL-CueO as a template to amplify two fragments. Next, using ClaI-mCueO-F (SEQ ID NO: 7) and HindIII-CueO-R (SEQ ID NO: 6), PCR was performed with a mixture of the two amplified fragments as a template to produce a DNA fragment with the mutated sequence for mutant CueO-D360S. The same conditions as in Experiment 2 of Example 1 were used for PCR. The resulting DNA fragment was digested with ClaI and HindIII, and the digested DNA fragment was inserted into the ClaI-HindIII gap of the plasmid pKL-CueO to construct a plasmid pKL-mCueO-D360S. The base sequences of the primers used are shown below.
ClaI-mCueO-F (SEQ ID NO: 7):
   5'-TGGGGTATCGATGATGTTCCGGTGATCG-3'
mCueO-R1S (SEQ ID NO: 24): Underlined positions indicate introduced mutations.
   5'-GCATCCCCATCATCGAGAGCATCGGGTCC-3'
mCueO-F1S (SEQ ID NO: 25): Underlined positions indicate introduced mutations.
   5'-GGACCCGATGCTCTCGATGATGGGGATGC-3'
HindIII-CueO-R (SEQ ID NO: 6):
   5'-CCCAAGCTTTTATACCGTAAACCCTAACATCATCCCCGTATCTTC-3'

When G at position 304 was substituted, using the primers ClaI-mCueO-F (SEQ ID NO: 7) and mCueO-R2-N (SEQ ID NO: 15) and primers mCueO-F2-N (SEQ ID NO: 16) and HindIII-CueO-R (SEQ ID NO: 6), PCR was performed with pKL-mCueO-D360S as a template to amplify two fragments. Next, using ClaI-mCueO-F (SEQ ID NO: 7) and HindIII-CueO-R (SEQ ID NO: 6), PCR was performed with a mixture of the two amplified fragments as a template to produce a DNA fragment with the mutated sequence for mutant CueO-D360S/G304X. The same conditions as in Experiment 2 of Example 1 were used for PCR. The resulting DNA fragment was digested with ClaI and HindIII, and the digested DNA fragment was inserted into the ClaI-HindIII gap of the plasmid pKL-CueO to construct a plasmid for expression of D360S/G304X mutant.
ClaI-mCueO-F (SEQ ID NO: 7):
   5'-TGGGGTATCGATGATGTTCCGGTGATCG-3'
mCueO-R2-N (SEQ ID NO: 15) Underlined positions indicate introduced mutations (N is a mixture of A, T, G, and C):
   5'-GCAATCGCCATCNNCATCTGGCTGACC-3'
mCueO-F2-N (SEQ ID NO: 16) Underlined positions indicate introduced mutations (N is a mixture of A, T, G, and C):
   5'-GGTCAGCCAGATGNNGATGGCGATTGC-3'
HindIII-CueO-R (SEQ ID NO: 6):
   5'-CCCAAGCTTTTATACCGTAAACCCTAACATCATCCCCGTATCTTC-3'

Each plasmid constructed above was sequenced, and those confirmed to have the predetermined amino acid substitutions introduced were used in Experiment 2 below.

### (Experiment 2: Preparation of CueO Protein)

According to the conditions and procedure described in Experiment 2 of Example 2, each plasmid constructed and whose sequence was verified in Experiment 1 was introduced into an Escherichia coli BL21 strain to obtain recombinant Escherichia coli. The obtained recombinant Escherichia coli and the recombinant Escherichia coli BL21 (pKL-CueO) expressing wild-type CueO were cultured, and the amount of protein in the culture supernatants and the ABTS oxidation activity were measured.

The following table shows the ratio of the specific activity of the supernatant fluid obtained from the recombinant Escherichia coli by each plasmid for mutant expression to the specific activity (WT) of the supernatant fluid obtained from the recombinant Escherichia coli BL21 (pKL-CueO).

All D360S/G304X double mutants were confirmed to have ABTS oxidation activity exceeding that of wild-type WT. In particular, mut15 (D360S/G304V), mut16 (D360S/G304P), mut19 (D360S/G304T), mut20 (D360S/G304N), mut22 (D360S/G304R), and mut23 (D360S/G304H) had high activity. The amino acid sequences of mut14 to mut24 containing the signal peptides are shown in SEQ ID NO: 23.

**[Table 5]**

| | Mutation site | Fold (/WT) |
|---|---|---|
| WT | Wild type | 1.0 |
| mut14 | D360S/G304A | 1.4 |
| mut15 | D360S/G304V | 4.4 |
| mut16 | D360S/G304P | 4.8 |
| mut17 | D360S/G304L | 3.5 |
| mut18 | D360S/G304F | 1.9 |
| mut19 | D360S/G304T | 5.2 |
| mut20 | D360S/G304N | 5.5 |
| mut21 | D360S/G304C | 2.7 |
| mut22 | D360S/G304R | 5.1 |
| mut23 | D360S/G304H | 6.0 |
| mut24 | D360S/G304D | 3.0 |

## Claims

1. A protein comprising an amino acid sequence [1] or [2]:
[1] an amino acid sequence containing at least a region from position 29 to position 516 in an amino acid sequence of SEQ ID NO: 14 with mutations (a) and (b) introduced thereinto:
(a) a substitution of D at position 360 with an amino acid other than D; and
(b) one or more selected from a substitution of G at position 304 with an amino acid other than G, a substitution of D at position 373 with an amino acid other than D, and a substitution of Q at position 374 with an amino acid other than Q; or
[2] an amino acid sequence having 90% or more sequence identity to the amino acid sequence [1], the amino acid sequence having amino acid residues corresponding to positions 360, 304, 373, and 374 of the amino acid sequence of SEQ ID NO: 14 identical to those of the amino acid sequence [1], wherein
the protein having laccase activity.

2. The protein according to claim 1, wherein
the mutation (a) is a substitution of D at position 360 with K or S, and
the mutation (b) includes a substitution of G at position 304 with K, N, R, V, L, S, Y, C, D, F, P, T, A, or H.

3. The protein according to claim 2, wherein
the mutation (a) is a substitution of D at position 360 with K, and
the mutation (b) includes a substitution of G at position 304 with K, N, R, V, L, S, Y, C, D, or F.

4. The protein according to claim 3, wherein
the mutation (b) includes a substitution of G at position 304 with K, N, or R.

5. The protein according to claim 2, wherein
the mutation (a) is a substitution of D at position 360 with S, and
the mutation (b) includes a substitution of G at position 304 with V, P, T, N, R, H, A, L, F, C, or D.

6. The protein according to claim 5, wherein
the mutation (b) includes a substitution of G at position 304 with V, P, T, N, R, or H.

7. The protein according to any one of claims 2 to 6, wherein
the mutation (b) further includes a substitution of D at position 373 with A and a substitution of Q at position 374 with A.

8. The protein according to any one of claims 1 to 7, wherein
the amino acid sequence [2] includes a region identical to a region corresponding to position 299 to position 398 of the amino acid sequence of SEQ ID NO: 14 in the amino acid sequence [1].

9. A polynucleotide comprising
a base sequence encoding the amino acid sequence of the protein according to any one of claims 1 to 8.

10. A vector or a DNA fragment comprising the polynucleotide according to claim 9.

11. A transformed cell retaining the vector or the DNA fragment according to claim 10.

12. A method for oxidizing a laccase substrate compound, comprising
oxidizing the laccase substrate compound in a reaction solution containing the protein according to any one of claims 1 to 8 and the laccase substrate compound.

13. The method according to claim 12, not comprising
adding copper ions to the reaction solution.

14. A biofuel cell comprising
a cathode-side electrode containing the protein according to any one of claims 1 to 8.

15. The biofuel cell according to claim 14, further comprising:
an anode-side electrode; and
an electrolyte layer arranged between the cathode-side electrode and the anode-side electrode, copper ions being not added to the electrolyte layer.
